(19) Europäisches Patentamt
European Patent Office
Office européen des brevets

(11) Publication number: **0 374 991 A1**

# (12) EUROPEAN PATENT APPLICATION

(21) Application number: 89202967.9

(51) Int. Cl.5: **C07D 241/46, A61K 31/495**

(22) Date of filing: 22.11.89

(30) Priority: 23.11.88 IE 3496/88
24.07.89 IE 2383/89

(43) Date of publication of application:
27.06.90 Bulletin 90/26

(84) Designated Contracting States:
AT BE CH DE ES FR GB GR IT LI LU NL SE

(71) Applicant: WESTMART HILL LIMITED
6 Greenfield Crescent
Donnybrook Dublin 4(IE)

(72) Inventor: O'Sullivan, John Francis
"Glandore" Stradbrook Road
Blackrock County Dublin(IE)

(74) Representative: Smulders, Theodorus A.H.J., Ir. et al
Vereenigde Octrooibureaux Nieuwe Parklaan 107
NL-2587 BP 's-Gravenhage(NL)

## (54) New antimicrobial phenazine derivatives, method for their preparation, compositions containing them, and their use in therapy.

(57) The invention provides a new antimicrobial phenazine derivative which has the structural Formula I

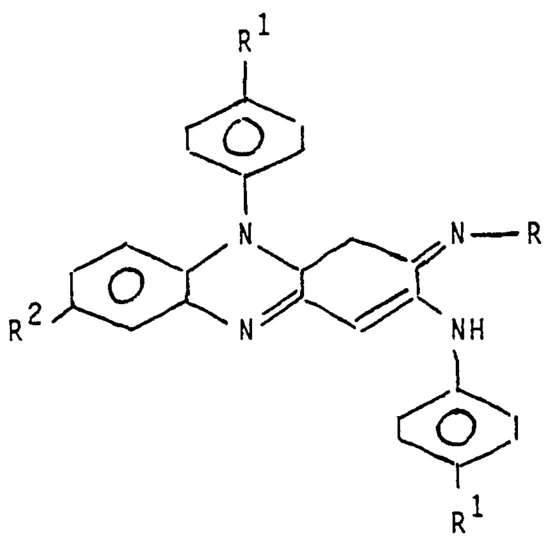

I

characterized by one of the following five sets (a - e) of values for R, $R^1$ and $R^2$

(a) R = $CH_3$- ; $R^1$ = H ; $R^2$ = Cl
(b) R = $CH_3$. $CH_2$-; $R^1$ = H ; $R^2$ = Cl
(c) R = $CH_3$. $CH_2$-; $R^1$ = Br; $R^2$ = H
(d) R = $CH_3$. $CH_2$-; $R^1$ = Cl; $R^2$ = Cl
(e) R = $CH_3$. $CH_2$-; $R^1$ = Br; $R^2$ = Cl

The last three compounds (Ic), (Id) and (Ie) are preferred. A method for preparing these compounds is also

EP 0 374 991 A1

provided, comprising

(1) reacting a substituted nitrobenzene (III) with a substituted and formylated aniline (IV) to produce a substituted 2-nitrodiphenylamine (V),

(2) reducing the product of Step (1) to form a 2-aminodiphenylamine (VI);

(3) oxidatively condensing the product of Step (2) to unite two molecules thereof forming a substituted 3-anilino-2-imino-10-phenyl-2,10-dihydrophenazine (VII); and

(4) reacting the product of Step 3 with methylamine or ethylamine.

The compounds are useful in the treatment of ailments caused by various named bacterial families and species, especially Mycobacterium avium and M leprae, and in the treatment of opportunistic infections in patients whose immune system is compromised.

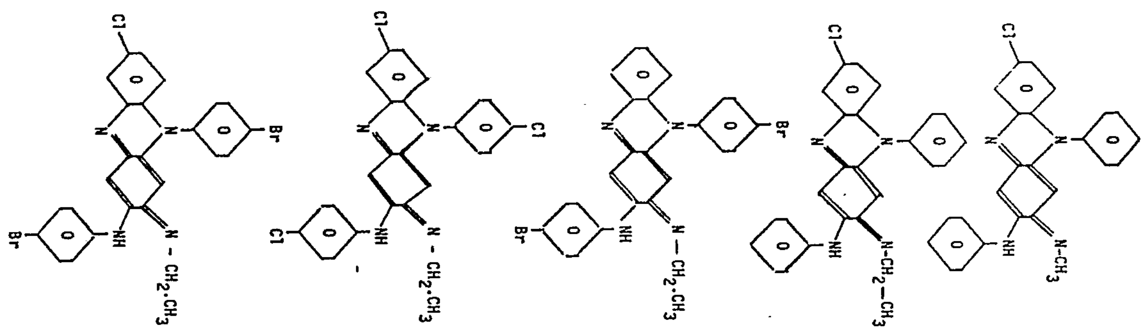

(Ie) (Id) (Ic) (Ib) (Ia)

## New antimicrobial phenazine derivatives, method for their preparation, compositions containing them, and their use in therapy.

This invention relates to five new phenazine derivatives, to methods for their production, to pharmaceutical compositions containing them as active ingredients, and to the use of said derivatives for preparing medicaments for the treatment of inflammation and for the therapy of certain microbial infections.

Owing to the development by pathogenic microorganisms of resistance to known curative agents there is a constant need for new antimicrobials. In the specific field of the phenazines, such as Clofazimine (3-p-Chloroanilino-10-(p-chlorophenyl)-2,10-dihydro-2-(iso-propylimino)phenazine) many compounds structurally related to Clofazimine have been produced and tested for activity against disease-causing bacteria, (see, for example O'Sullivan, Conalty and Morrison, J Med Chem 1988 31, 567).

The invention provides five new antimicrobial phenazine derivatives which have the structural Formula I

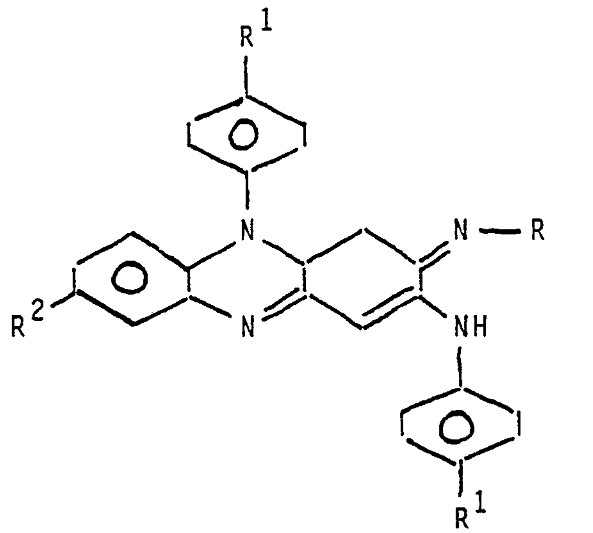

I

characterized by one of the following five sets (a - e) of values for R, $R^1$ and $R^2$

(a) R = $CH_3$- ; $R^1$ = H ; $R^2$ = Cl

(b) R = $CH_3.CH_2$-; $R^1$ = H ; $R^2$ = Cl

(c) R = $CH_3.CH_2$-; $R^1$ = Br; $R^2$ = H

(d) R = $CH_3.CH_2$-; $R^1$ = Cl; $R^2$ = Cl

(e) R = $CH_3.CH_2$-; $R^1$ = Br; $R^2$ = Cl

The invention also provides a method for preparing the above compounds characterized by

(1) reacting an appropriately substituted nitrobenzene (III) with an appropriately substituted and previously formylated aniline (IV) to form a substituted 2-nitrodiphenylamine (V);

(2) reducing the product of Step (1) to form the corresponding 2-aminodiphenylamine (VI);

(3) oxidatively condensing the product of Step (2) whereby two molecules of said product unite to form a substituted 3-anilino-2-imino-10-phenyl-2,10-dihydrophenazine (VII); and

(4) reacting the product of Step (3) with methylamine or ethylamine to obtain the required phenazine derivative of Formula I, wherein R is methyl when methylamine has been used, and R is ethyl when ethylamine has been used for this reaction.

The Roman numerals in parentheses are those used to identify the respective structural formulae in the accompanying drawings.

Step (1) is preferably performed in the presence of anhydrous potassium carbonate in boiling dimethylformamide.

A suitable reducing agent for Step (2) is zinc with acetic acid.

Step (3) is preferably carried out using ferric chloride solution, again preferably in an acetic acid or ethyl alcohol medium.

Step (4) is preferably performed by refluxing in dioxan (which commonly takes about four hours), or by autoclaving in ethanol at 100-110° C.

Phenazine derivatives, such as Clofazimine (mentioned above) are now well established as having both

antimicrobial and antiinflammatory activity. The compounds of the present invention, however, surprisingly show a much stronger antimicrobial activity than is commonly encountered among phenazine derivatives, and a wider spectrum of pathogenic organisms against which they are effective. They also have a strong antiinflammatory activity. The last three compounds listed above (Ic, Id and Ie) have particularly desirable properties in those respects.

The antimicrobial activity of the previously known phenazines, such as Clofazimine, appears to depend on the production of activated oxygen such as native peroxide and superoxide by a redox cycling mechanism. This catalytic oxidant effect is attended with a corresponding antimicrobial activity.

Although the present invention is not bound to or limited by any theory of how it acts, it can be stated that on administration, the compounds of the invention unexpectedly produce what appears to be a greatly increased production of superoxide from FMLP-peptide stimulated neutrophils, and the resultant antimicrobial activity parallels this activity. The peptide (FMLP) employed in neutrophil stimulation is N-Formyl-L-methionyl-L-leucyl-L-phenylalanine. These phenazine compounds have enhanced activity over previously known phenazines against, for example, Mycobacterium avium, M tuberculosis, M ulcerans, M leprae, M intracellulare and M pneumoniae. They may also have enhanced activity against discoid lupus erythematosus, and rheumatoid arthritis. In particular, Id, a trichloro compound, and Ic, a dibromo compound, exhibit a pronounced activity against Mycobacterium avium.

Furthermore these new compounds are active against strains of the families Corynebacteriaceae, Bacillaceae, Micrococcaceae, Actinomycetaceae and Streptomycetaceae.

Among conditions yielding to topical treatment with these new compounds were bedsores and burns, animal bites and abscesses. In the veterinary field inflamed joints, lesions, ulcerations and stiffness also respond well to topical treatment with the compounds.

The compounds are therapeutically useful against opportunistic infections, especially in those patients whose immune system is compromised by Acquired Immune-deficiency Syndrome (AIDS).

The invention will be appreciated in greater detail from the description given in the following specific examples, wherein Roman numerals follow the names of compounds identified or generically designated by structural formula in the accompanying drawings.

Example 1.

2-imino precursor of a compound I wherein $R^1 = H$, $R^2 = Cl$. (a) 4-Chloro-2-nitrodiphenylamine (V).

10.5 kg 2,5-Dichloronitrobenzene (III) was dissolved in 24 l aniline containing 5.25 kg anhydrous sodium acetate in a 50 l glass reaction vessel with a bottom outlet. The mixture was agitated at reflux temperature (185 °C approximately) for 40 hours at atmospheric pressure. At the end of this reflux period the mixture was distilled under slightly reduced pressure (as required to produce a steady distillation) until 14 l aniline had been collected. The contents of the vessel were now cooled to 80 °C, acidified with hydrochloric acid to a pH of 1-2 and stirred for 20 minutes, after which stirring was discontinued. There were now found to be two layers in the mixture. The darker lower layer (containing the product) was run off from the aniline hydrochloride solution of the top layer which latter was retained in the vessel.

The product solidified from the lower layer on cooling it to below 45 °C.

The top layer was repeatedly extracted with chloroform to recover any residual product. Such product was obtained by evaporation of the solvent.

The yield was of the order of 13 kg. This product was purified by means of 40 l ethanol by boiling at 78 °C for 1 hour. The mixture was then cooled to ambient temperature with stirring, causing the purified product to crystallize out. The product was filtered off and washed twice with 5 l portions of ethanol. It was then dried in an oven at a temperature not above 30 °C to give a yield of approximately 11 kg or 78% of theory. The product was dark red in colour and had a melting point of 143-145 °C.

(b) 4-Chloro-2-aminodiphenylamine (VI).

The product (V) of step (a) was reduced by treating it with zinc in acetic acid as follows:

In a clean dry 100 l Pfaudler vessel 45 l glacial acetic acid was charged through the cover mouth or charging hole. The stirrer was started and 16.5 kg zinc powder was added. The temperature was raised to 40 °C and kept between 40 and 45 °C by cold water cooling, while 10.5 kg of the pure 4-Chloro-2-nitrodiphenylamine (prepared in step (a)) was added in small portions over a period of 1 to 2 hours. Care was taken to avoid too high a temperature rise, certainly not above 55° C. After the addition was complete the mixture was stirred at 50-55° C for 30 minutes or until the initial red colour had changed to dark green (if necessary extra zinc powder may be added to achieve this). Then 40 l water was added and the mixture was cooled down to 10-15° C. The product precipitated out with zinc salts and, after filtering, washing and drying at 40-45° C the product was recrystallized from ethanol to give approximately 8.26 kg of a solid of melting point 113-116° C.

(c) 3-Anilino-7-chloro-2-imino-10-phenyl-2,10-dihydro-phenazine (VII).

3 kg of the product of step (b) was dissolved in 40 l ethanol containing 9 kg $FeCl_3.6H_2O$ and 500 ml concentrated HCl was added with stirring at ambient temperature (but not above 20°, preferably about 15° C). Stirring was continued for 5 hours and the mixture was then allowed to stand overnight. The melting point of the hydrochloride obtained is indefinite with decomposition from 260° C and upwards. The yield of the title compound was 2.7 kg or 90% of theory; it had a melting point of 186-187° C.

Example 2.

3-Anilino-7-chloro-2-methylimino-10-phenyl-2,10-dihydro-phenazine (Ia)

2.2 kg of the product of Example 1 was dissolved in 75 l dioxan in a 200 l glass reaction vessel fitted with an agitator and reflux condenser and with a heating mantle. 25 l of a 25% aqueous solution of methylamine was added and the mixture refluxed with agitation for 5 hours. The product was recrystallized from a dioxan/water/ethanol mixture to give a yield of 69% of theory.
Empirical formula $C_{25}H_{19}N_4Cl$. Molecular weight 410.5

| Analysis | C | H | N | Cl |
|---|---|---|---|---|
| Required | 73.1 | 4.6 | 13.6 | 8.6% |
| Found | 73.1 | 4.4 | 13.4 | 8.4% |

Mp 203° C (decomposition).
Thin layer chromatography was used to verify the purity of the reagents. (Merck Silica Gel (Art 7736), developed with 5-10% methanol in chloroform).

Example 3

3-Anilino-7-chloro-2-ethylimino-10-phenyl-2,10-dihydro-phenazine (Ib)

3 kg of the product of Example 1 was dissolved in 60 l dioxan in a 200 l vessel fitted with reflux condenser, agitator and heating mantle. 14 l of a 70% solution of monoethylamine in water was added and the whole refluxed with agitation for at least 5 hours. The product was recrystallized from a dioxan/water/ethanol mixture to give the cited compound in a 78% yield.
Empirical formula $C_{26}H_{21}N_4Cl$. Molecular weight 424.5

| Analysis | C | H | N | Cl |
|---|---|---|---|---|
| Required | 73.4 | 4.9 | 13.2 | 8.4% |
| Found | 73.1 | 5.0 | 12.9 | 8.3% |

Mp 180-182° C with decomposition.

Example 4

Activity of the Example 2 compound against Mycobacterium smegmatis, M avium and M leprae.

Bactericidal activity was detected at 2 µg per ml or less.

Example 5

Activity of the Example 3 compound against selected bacteria in vitro.

Mycobacterium smegmatis, M avium and M leprae.
Activity was detected at concentrations of 2 µg per ml or less.

Example 6

7-Chloro-3-p-chloroanilino-2-ethylimino-10-p-chlorophenyl-2,10-dihydrophenazine (Id)

p-Chloroaniline was formylated.
The product was treated with anhydrous $K_2CO_3$ in DMF and 2,5-Dichloronitrobenzene was added. The mixture was heated under reflux for 8 hours. The product (obtained in 50-60% yield) was then reduced and the reduction product subsequently oxidized to yield Trichloroanilino aprosafranine hydrochloride (TAAS).
5g of TAAS, dissolved in dioxan and 20 ml of a 70% aqueous solution of ethylamine, was heated under reflux for 4.5 hours. The reaction mixture was then filtered and the flask washed out with ethanol. Dilution of the filtrate with water caused precipitation of the required product, which after recrystallization from dioxan/ethanol/water gave a yield of 3.8g (77% of theory) of a compound having a melting point of 184° C with decomposition.
Elementary analysis based on the molecular formula $C_{26}H_{19}N_4Cl_3$ (MW 493.5) showed:

| | | | | |
|---|---|---|---|---|
| Calculated: | C: 63.2 | H: 3.9; | N: 11.3; | Cl: 21.6 (%) |
| Found: | 62.7; | 3.8; | 11.1; | 21.5. |

Example 7

3-p-Bromoanilino-10-p-bromophenyl-2-ethylimino-2,10-dihydrophenazine (Ic)

p-Bromoaniline was formylated.
The product was condensed with 2-Fluoronitrobenzene to give 4′-Bromo-2-nitrodiphenylamine. The latter was then reduced to the 2-amino compound and oxidized with $FeCl_3$ solution to give the anilino-aposafranine having bromine in the p-position of the phenyl rings.
This "parent" compound (actually 2.1 g of the hydrochloride) was heated for 4.5 hours in 70 ml dioxan with 20 ml of 70% aqueous ethylamine solution, and yielded 1.4g of the desired product (68% of theory). Mp indef 180° up, when crystallized from dioxan/ethanol/water.
Elementary analysis based on the molecular formula $C_{26}H_{20}N_4Br_2$ (MW 548) showed:

| Calculated: | C: 56.9; | H: 3.6; | N: 10.2; | Br: 29.2 (%) |
|---|---|---|---|---|
| Found: | 56.9; | 3.8; | 10.0; | 29.2. |

Example 8

3-p-Bromoanilino-10-p-bromophenyl-7-chloro-2-ethylimino-2,10-dihydrophenazine (Ie)

This compound, prepared in an analogous manner, had Mp 186 °C dec when crystallized from dioxan/ethanol/water. Elementary analysis based on the molecular formula $C_{26}H_{19}N_4Br_2Cl$ (MW 582.5) showed:

| Calculated: | C: | H: | N: | Br: | Cl: |
|---|---|---|---|---|---|
| | 53.6; | 3.3; | 9.6; | 27.5; | 6.1 (%) |
| Found: | 53.6; | 3.1; | 9.8; | 27.9. | 5.7 |

The compounds of the invention may be made up in conventional fashion into pharmaceutical formulations which may be for example, tablets, capsules, injections, ointments, powders, suppositories or sprays. Ointments, powders and other preparations for topical application are preferred. It has been found that the addition of vegetable oil to such preparations improves their absorption through the skin. A suitable vegetable oil is coconut oil.

## Claims

1. A new antimicrobial phenazine derivative which has the structural formula I

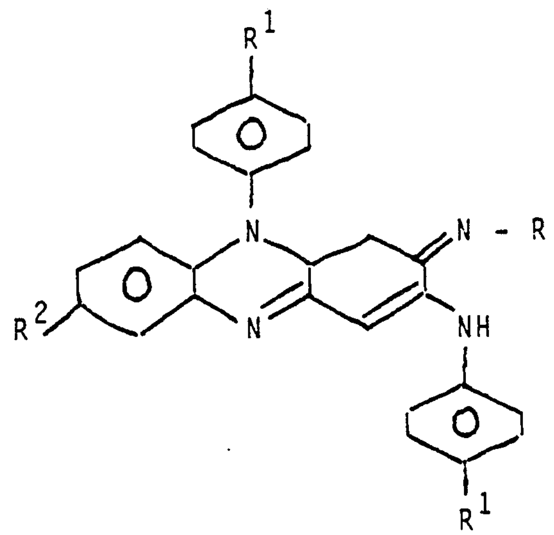

I

characterized by one of the following five sets (a - e) of values for R, $R^1$ and $R^2$

(a) R = $CH_3$- ; $R^1$ = H ; $R^2$ = Cl
(b) R = $CH_3.CH_2$-; $R^1$ = H ; $R^2$ = Cl
(c) R = $CH_3.CH_2$-; $R^1$ = Br; $R^2$ = H
(d) R = $CH_3.CH_2$-; $R^1$ = Cl; $R^2$ = Cl
(e) R = $CH_3.CH_2$-; $R^1$ = Br; $R^2$ = Cl

2. A compound according to Claim 1, characterized by one of sets (c) , (d) and (e) of said values.

3. A method for preparing a compound claimed in Claim 1 or 2, characterized by performing the following method steps:-

(1) reacting an appropriately substituted nitrobenzene (III) with an appropriately substituted and previously formylated aniline (IV) to produce a substituted 2-nitrodiphenylamine (V);

(2) reducing the product of Step (1) to form the corresponding 2-aminodiphenylamine (VI);

(3) oxidatively condensing the product of Step (2) whereby two molecules of said product unite to form a substituted 3-anilino-2-imino-10-phenyl-2,10-dihydrophenazine (VII); and

(4) reacting the product of Step (3) with methylamine or ethylamine to obtain the required phenazine derivative of Formula I, wherein R is methyl when methylamine has been used, and is ethyl when ethylamine has been used for this reaction.

4. A method according to Claim 3, characterized by performing Step (1) in the presence of anhydrous potassium carbonate in boiling dimethylformamide.

5. A method according to Claim 3 or 4, characterized by using ferric chloride solution for Step (3).

6. A method according to any of Claims 3 to 5, characterized by performing step (3) in an acetic acid or ethyl alcohol medium.

7. A method according to any of Claims 3 to 6, characterized by performing step (4) by refluxing in dioxan, or by autoclaving in ethanol at 100 - 110° C.

8. A pharmaceutical composition characterized by a content, as active ingredient, of a compound claimed in claim 1 or 2.

9. A method of preparing a medicament for use in the treatment of an ailment caused or exacerbated by any of the following microorganism species:
Mycobacterium avium
M tuberculosis
M ulcerans
M leprae
M intracellulare
M pneumoniae
or by a member species of one of the following families of microorganisms
Corynebacteriaceae
Bacillaceae
Micrococcaceae
Actinomycetaceae
Streptomycetaceae
characterized by the use of a compound claimed in Claim 1 or 2 as active ingredient.

10. A method of preparing a medicament for use in the treatment of an opportunistic infection in a patient whose immune system is compromised, characterized by the use of a compound claimed in Claim 1 or 2 as active ingredient.

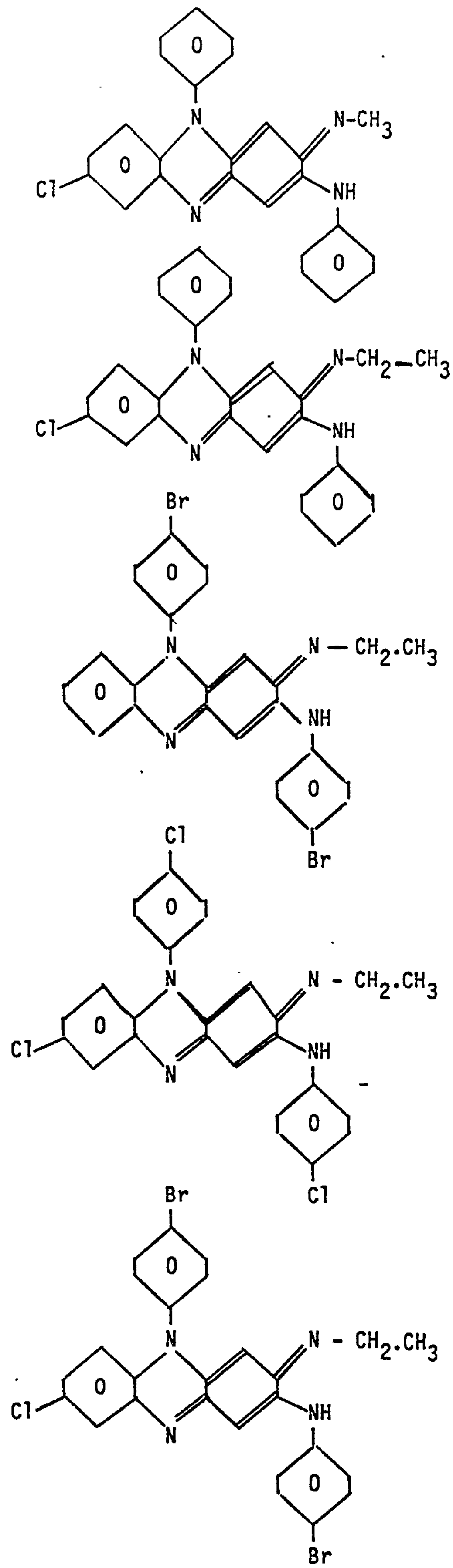

(Ia)

(Ib)

(Ic)

(Id)

(Ie)

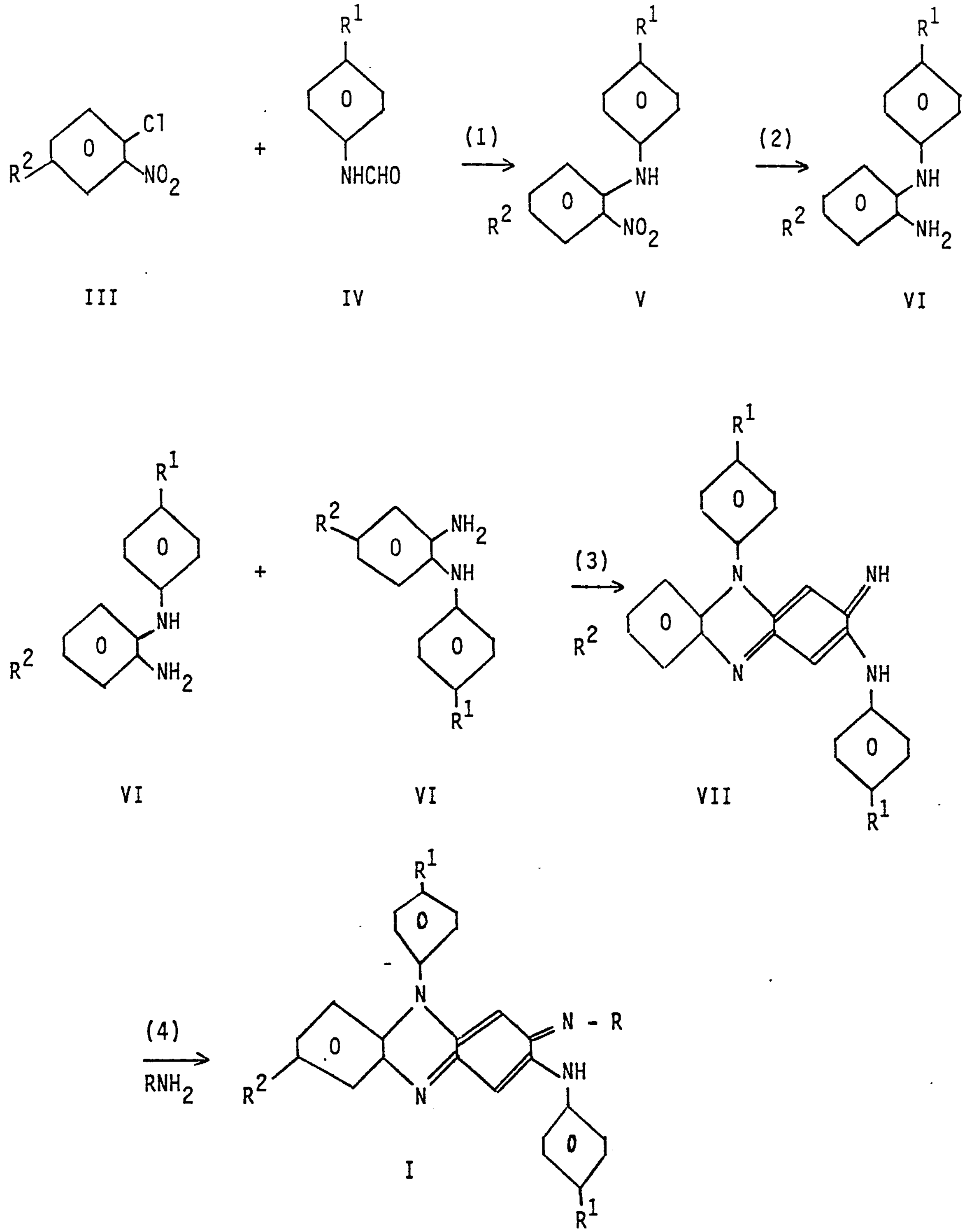


III IV V VI

VI VI VII

I

European Patent Office

# EUROPEAN SEARCH REPORT

Application Number

EP 89 20 2967

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | JOURNAL OF MEDICINAL CHEMISTRY, vol. 31, no. 3, March 1988, page 567, The American Chemical Society; J.F. O'SULLIVAN et al.: "Clofazimine analogues active against a clofazimine-resistant organism" * Whole document * | 1,3,8,9 | C 07 D 241/46<br>A 61 K 31/495 |
| A | FR-M- 7 925 (MAY AND BAKER LTD) * Claims * | 1,8,9 | |
| A | FR-A-1 174 378 (J.R. GEIGY) * Claims; table I * | 1,8,9 | |
| A | US-A-3 092 549 (A.G. GIRARD) * Columns 1,2; claims * | 1,3,8,9 | |
| A | US-A-2 943 089 (V.C. BARRY et al.) * Claims * | 1,8,9 | |

TECHNICAL FIELDS SEARCHED (Int. Cl.5)

C 07 D 241/00
A 61 K 31/00

The present search report has been drawn up for all claims

| Place of search | Date of completion of the search | Examiner |
|---|---|---|
| THE HAGUE | 28-02-1990 | DE JONG B.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)